# EUROPEAN PATENT APPLICATION

(11) **EP 0 995 556 A2**
(43) Date of publication of application: **26.04.2000**
(21) Application number: 98204334.1
(22) Date of filing: 21.12.1998
(51) Int. Cl.: B25J 9/16

(54) **A system for dynamic evaluation of human behavior**

(30) Priority: 22.09.1998 NL 1010150
(71) Applicant: Even-Zohar, Oshri, 1013 BG Amsterdam (NL)
(72) Inventor: Even-Zohar, Oshri, 1013 BG Amsterdam (NL)

(57) **Abstract**

The invention is a system that combines motion capture technologies and simulation technology, it creates a new measurement and correction tool for determining. registering and evaluating human functional performance to a range of given situations.

The invention creates a new principal in simulations where human behavior needs to be evaluated and / or improved this principal establishes a way to achieve a simulation state whereby the person(s) involved are affecting by their behavior, the physical and the visual.

## Description

The invention creates a new principal in simulations where human behavior needs to be evaluated and / or improved this principal establishes a way to achieve a simulation state whereby the person(s) involved are affecting by their behavior, the physical and the visual environments they are immersed in.

The invention is a system that combines motion capture technologies and simulation technology, the invention is a combination of commercially available hardware / software elements and a new authoring and control software environment.

The invention creates a new measurement and correction tool bearing applications in the industries listed below. The invention creates the possibility of immersing one or more people in a fully reactive virtual and physical environments for determining, registering and evaluating human functional performance to a range of given situations. In the context of either functional training situations, or forms of immerse / reactive types of experiences.

### Description of the new simulation principal.

A person(s) is on an N-degrees Motion base (hydraulic or other). Positional and rotational data of the person(s) full body is transmitted to a fast computer at a rate above 25 frames per second. The data generated by the person(s) motions is sent to a fast processing unit, then mixed and partially sent back to the motion base.

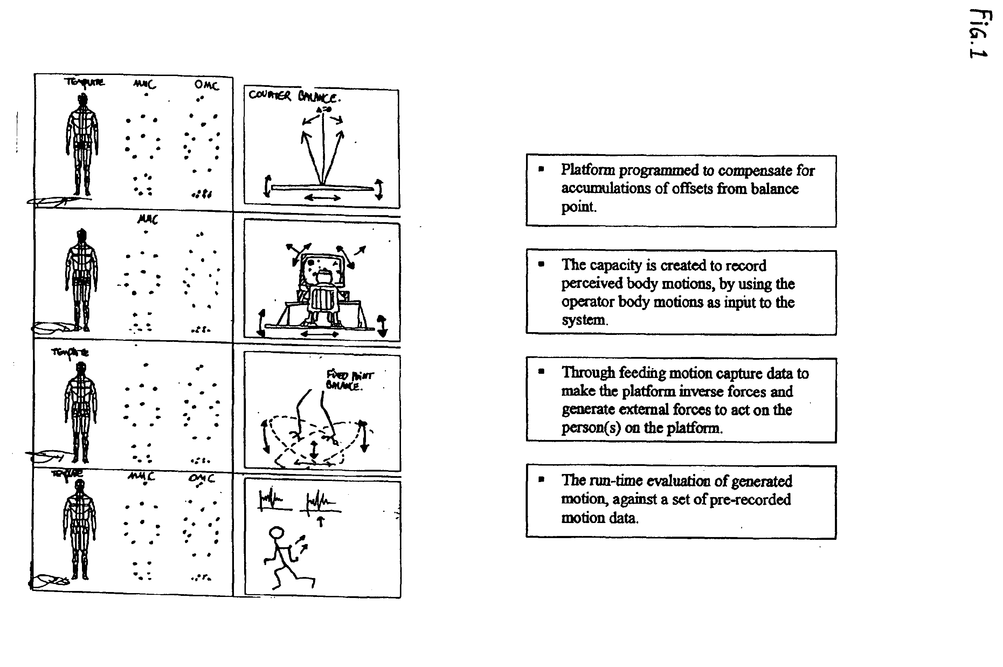

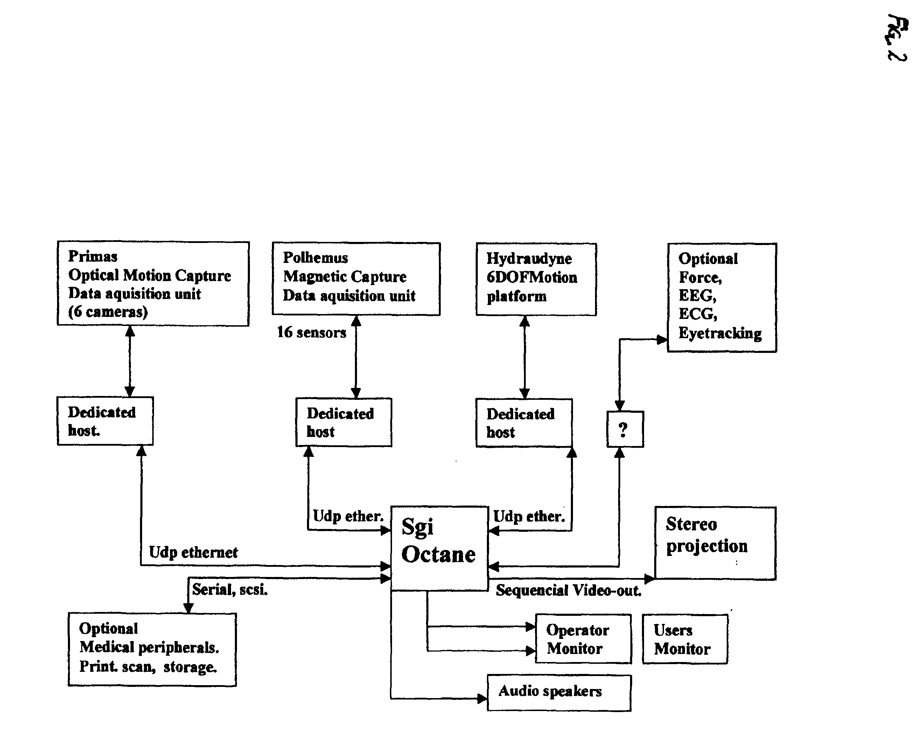

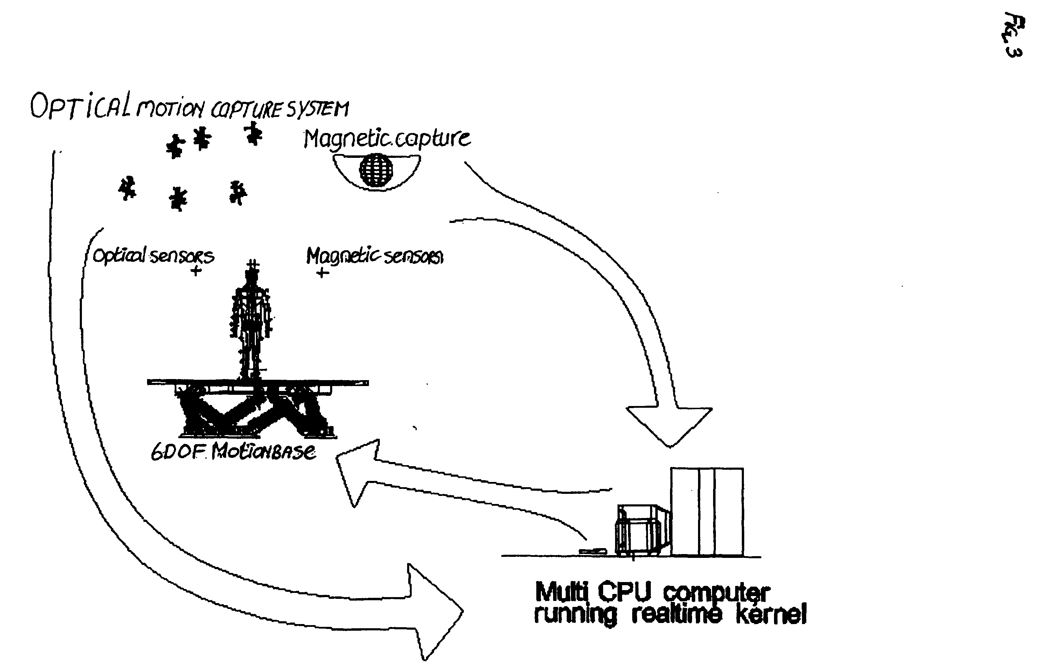

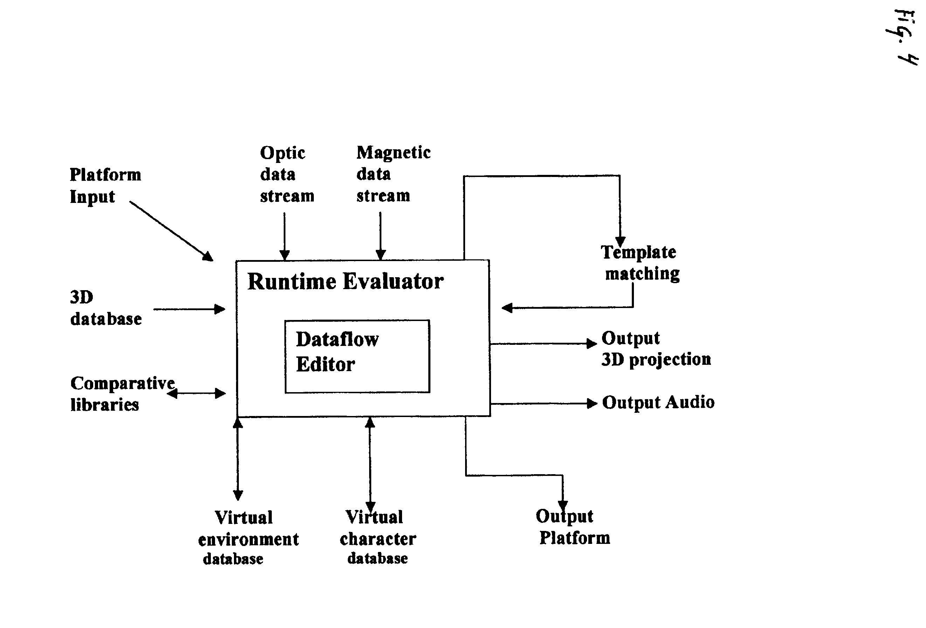

These processes are running at speeds above 25fps (frames per second). The system responds to the person(s) accumulating offsets of equilibrium, either through correction of through amplifications (positive or negative vector modifications). The response of the N-DOF Motion base is application dependent, and is fully user configurable through the use of the runtime kernel user interface. (Fig.1).

### Difference from current state of simulation technologies.

With current simulation technologies, measurements are taken of person(s) response time to a given simulated situation, there is no data referring to the spatial behavior of the person(s) in the simulation. At the core of the new simulation principal is the feedback loop created when data generated from the person(s) motions, influences the simulation. A full data set of the spatial behavior of the person(s) motions is registered 3 and compared in real-time with the perceived optimal behavior.

The simulation conditions are depending on the person(s) activities. The instructor gets information not only about the response time to a given simulation scenario, but also the reasons why the response took the time it took. This bears direct application on ergonomic spatial design.

### Industrial applications:

**Simulation ,** to be divided into

**Aviation and space research.**
An application that allows the registration of pilots and astronauts spatial coordination reaction times and latency, in real-time, while responding to an array of given simulated situations.

**Automotive.**
An application that records and displays in real-time the spatial trajectories of the driver movements, and creates a 3D database of those movements. The application generates data relating to a better ergonomic prototyping and design of driving environments.

**Leisure and entertainment.**
An application that records rides in real-time.

**Robotics and telemetry.**
An application that creates a telemetric reactive environment allowing the driving of remotes in a fully interactive manner. The operator is receiving motion feedback from the remote terrain.

**Medical.**
An application that enables person(s) to react to offsets in their own equilibrium, through reduction or amplification of their own motions feedback to the Motion base they are on.

**Architecture.**
An application for monitoring ground reaction forces in earthquake sensitive buildings.

## Claims

1. The first claim is relating to the workmethods that make use of a real-time feedback loop where a person(s) generate 3D motion data which causes the environment to react and the person(s) to generate new 3D motion data.

2. The claim is for simulation systems that combine full or partial body **motion - capture** systems in combination with **Motion platforms,** whereby the data generated from the capture systems influences **in real-time** the motions of the Platforms.

3. The claim is for a method for recording 3D simulation rides in real-time, whereby the motions of the camera through a virtual environments are generated by a person(s) motion capture data.
